Europäisches Patentamt

European Patent Office

Office européen des brevets

(11) Veröffentlichungsnummer: **0 045 008**
**B1**

(12) **EUROPÄISCHE PATENTSCHRIFT**

(45) Veröffentlichungstag der Patentschrift:
17.12.86 .

(21) Anmeldenummer: 81105543.3

(22) Anmeldetag: 15.07.81

(51) Int. Cl.⁴: **B 01 F 3/08,** B 01 F 3/22,
A 61 K 7/00, A 61 K 9/10

(54) Verfahren zur Herstellung von pharmazeutischen und kosmetischen Dispersionen.

(30) Priorität: 24.07.80 DE 3028005

(43) Veröffentlichungstag der Anmeldung:
03.02.82 Patentblatt 82/5

(45) Bekanntmachung des Hinweises auf die Patenterteilung:
17.12.86 Patentblatt 86/51

(84) Benannte Vertragsstaaten:
AT BE CH DE FR GB IT LI NL SE

(56) Entgegenhaltungen:
DE-A-2 018 092
FR-A-1 498 294
US-A-1 347 734
US-A-1 882 834
US-A-2 254 049
US-A-3 579 461
US-A-3 635 834

Die Akte enthält technische Angaben, die nach
dem Eingang der Anmeldung eingereicht wurden
und die nicht in dieser Patentschrift enthalten sind.

(73) Patentinhaber: BAYER AG, Konzernverwaltung RP
Patentabteilung, D-5090 Leverkusen 1 Bayerwerk
(DE)

(72) Erfinder: Bücheler, Manfred, Ing. Grad.,
Lorkenhoehe 49, D-5063 Overath (DE)
Erfinder: Klinksiek, Bernd, Ing. Grad.,
Obervolbach 10, D-5060 Bergisch- Gladbach 4 (DE)
Erfinder: Gehringer, Hans, H;inering 45, D-5000
Köln 71 (DE)
Erfinder: Schnöring, Hildegard, Dr., Rappenweg 8,
D-5600 Wuppertal 11 (DE)

LIBER, STOCKHOLM 1986

EP 0 045 008 B1

## Beschreibung

Die Erfindung betrifft ein Verfahren zur Herstellung von feinteiligen, stabilen, pharmazeutischen und kosmetischen Dispersionen aus einer wäßrigen Phase und einer in Wasser unlöslichen oder nicht vollständig löslichen organischen Phase.

Üblicherweise werden pharmazeutische und kosmetische Emulsionen so hergestellt, daß man die bei Temperaturen von 60 bis 70°C aufgeschmolzene Fettphase oder organische Phase und die gesamte auf die gleiche Temperatur gebrachte wäßrige Phase in einem Rührkessel vereinigt und voremulgiert und die so erhaltene Rohemulsion in einem Mantel- oder Durchflußkühler auf 20 bis 40°C abkühlt und anschließend mit einem Hochdruckhomogenisator feinstdispergiert.

Nachteilig bei diesem Verfahren ist, daß der gesamte Ansatz zunächst aufgeheizt und nach dem Voremulgieren notwendigerweise wieder abgekühlt werden muß, um dann die gesamte Emulsion mit oft nur geringer disperser Fettphase mittels Hochdruckhomogenisatoren auf die gewünschte Feinheit zu bringen. Eine solche energieaufwendige Temperaturführung ist notwendig, da bei der Vergrößerung der Oberfläche der dispersen Phase durch die Homogenisierung eine ausreichende Stabilität der Emulsion bei erhöhter Temperatur unter gleichzeitiger mechanischer Beanspruchung nicht gegeben ist. Das Erzwingen der Temperatur- und Scherbeanspruchungsstabilität durch Emulgatoren ist nicht zulässig, da Menge und Art des Emulgatorsystems nach pharmakologischen Gesichtspunkten und denen der Lagerstabilität des Endproduktes, nicht aber an die weit höheren Stabilitätsansprüche während der Fertigung anzupassen sind.

Der Erfindung liegt die Aufgabe zugrunde, ein Verfahren zur Herstellung von feinteiligen, stabilen, pharmazeutischen und kosmetischen Dispersionen aus einer wäßrigen Phase und einer in Wasser unlöslichen oder nicht vollständig löslichen organischen Phase zu entwickeln, das bei gleicher Ausbeute eine günstigere Energiebilanz aufweist.

Diese Aufgabe wird erfindungsgemäß dadurch gelöst, daß man eine Phasenumkehr während der Dispergierung bei 40 bis 180°C in nur 5 bis 40 Vol.-% der äußeren Phase der fertigen Dispersion unter Verwendung von Hochdruck-, Ultraschall- oder Düsenhomogenisatoren oder Rotor/Statordispergiermaschinen und unter Beibehaltung der Konzentrationen von wäßriger und organischer Phase erreicht.

Dabei geht man so vor, daß man erst auf eine Teilchengröße von 0,1 bis 10 µm dispergiert und dann ohne Veränderung der Teilchengröße mit 60 bis 95 Vol.-% der äußeren Phase verdünnt, deren Temperatur unter dem Erstarrungspunkt der inneren Phase liegt. Die innere Phase wird auch als disperse und die äußere Phase als kontinuierliche Phase bezeichnet. Bei kosmetischen Dispersionen ist im allgemeinen die äußere (kontinuierliche) Phase die wäßrige und die innere (disperse) Phase die Fettphase. Das erfindungsgemäße Verfahren beruht also darauf, daß zunächst Emulsionskonzentrate mit sehr hohem Anteil an disperser Phase bei höheren Temperaturen hergestellt werden und die einmal erreichte Teilchengrößenverteilung durch Einleiten des Emulsionskonzentrats in die kalte restliche wäßrige Phase eingefroren wird. Dabei wurde überraschenderweise gefunden, daß die Feinheit dieser Emulsionskonzentrate besser oder zumindest ebenso gut ist wie die Feinheit der mit den üblichen Verfahren hergestellten niedrig konzentrierten Emulsionen. Dies steht im Widerspruch zu der als gesichert geltenden Lehrmeinung, daß die erreichbare Feinheit mit wachsender Volumenkonzentration an disperser Phase abnimmt (z.B. A. Mersmann, H. Grossmann: Dispergieren im flüssigen Zweiphasensystem, in Verfahrenstechnische Fortschritte beim Mischen, Dispergieren und bei der Wärmeübertragung in Flüssigkeiten, Preprints, GVC im VDI, Düsseldorf, Dezember 1978, S. 141/155).

Erfindungsgemäß werden die Temperatur und das Volumenverhältnis der beiden Phasen so eingestellt, daß während der Dispergierung eine Phasenumkehr erfolgt. Wird nach dem oben beschriebenen Verfahren z. B. die wäßrige Phase mit einem Anteil von 5 bis 40 % in die gesamte Fettphase eingeleitet und dispergiert, so bildet die wäßrige Phase zunächst die innere (disperse) Phase, in der äußeren (kontinuierlichen) Fettphase. Abhängig von Temperatur und Volumenverhältnis der beiden Phasen erfolgt dann während der Dispergierung eine Phasenumkehr, derart, daß die wäßrige Phase in die äußere Phase und die Fettphase in die innere Phase überführt wird. Die zu einem bestimmten Volumenverhältnis gehörende Phasenumkehrtemperatur läßt sich ohne weiteres empirisch ermitteln. Mit Hilfe des Phasenumkehrverfahrens lassen sich natürlich auch Wasser in Öl-Zubereitungen herstellen.

Aus US-PS-3 579 461 ist ein Verfahren zur Herstellung von O/W-Emulsionen bekannt, das wie folgt verläuft:

1. Herstellung einer W/O-Emulsion in einem Rührkessel durch Einmischen eines Teiles der Wasserphase in die vorgelegte komplette Ölphase;

2. Phasenumkehr zur O/W-Emulsion *durch Erhöhung der Konzentration der hydrophilen Phase oder durch Zugabe hydrophiler Emulgatoren bzw. anderer Dispergierhilfsmittel im Rührkessel.*

Demgegenüber stellt sich das erfindungsgemäße Verfahren wie folgt dar:

1. Herstellung einer W/O-Emulsion in einem Rührkessel durch Einmischen eines Teiles der Wasserphase in die vorgelegte komplette Ölphase;

2. Phasenumkehr zur O/W-Emulsion durch Passage der W/O-Emulsion durch ein hochenergisches Scherfeld einer Hochdruck-, Ultraschall-, Düsen- oder Rotor/Stator-Dispergiermaschine *ohne Veränderung der Phasenkonzentrationen* und ohne Zugabe von Emulgatoren bzw. anderer Dispergiermittel.

Mit der Erfindung sind folgende Vorteile verbunden:

1. Mit dem neuen Verfahren wird eine hohe Raum/ZeitAusbeute bei günstiger Wärmebilanz erreicht. Dies gilt insbesondere für Dispersionen mit geringem Anteil an disperser Phase/innere Phase), weil nur 5 bis 40 % der kontinuierlichen Phase (äußere Phase) an dem eigentlichen Emulgiervorgang beteiligt sind. Die dem letzten Verfahrensschritt entsprechende Verdünnung mit der kalten äußeren Phase sorgt gleichzeitig für die Abkühlung der Emulsion. Spezielle Wärmeaustauscher (Kühler) können entfallen. Die verbesserte Ausbeute führt in der Praxis bei einer gegebenen Anlage entweder zu kürzeren Taktzeiten oder zur 2- bis 5-fachen Menge Endprodukt, wenn man die Verdünnerkesselkapazität entsprechend erweitert.

2. Das Verfahren hat ferner den Vorteil, daß scher- und temperaturempfindliche Zusatzstoffe nicht geschädigt werden, wenn sie erst im letzten Schritt bei der Verdünnung mit der kalten äußeren Phase zugegeben werden. In diesem Stadium wird die Emulsion nicht mehr thermischen und mechanischen Belastungen ausgesetzt.

3. Da das Verfahren so durchgeführt wird, daß bei der Dispergierung eine Phasenumkehr eintritt, so daß die organische Phase zur inneren Phase wird, kann die Scherbeanspruchung des Produktes beim Dispergieren klein gehalten werden. Auf diese Weise werden kolloidale Strukturen und hochmolekulare Bestandteile in der organischen Phase weniger stark zerstört.

Im folgenden wird das erfindungsgemäße Verfahren anhand einer schematischen Zeichnung und anhand von Beispielen näher erläutert.

1. Allgemeine Zusammensetzung der Dispersionen

Die *wäßrige Phase* besteht aus einer wäßrigen Lösung von Glycerin, Glykol, kosmetischen oder pharmazeutischen Wirkstoffen, viskositätserhöhenden Zusätzen und Konservierungsmitteln.

Die *organische*, in Wasser unlösliche oder nicht vollständig lösliche *Phase* besteht aus Glycerin- oder Fettsäureester und/oder flüssigen oder halbfesten Kohlenwasserstoffen, höherwertigen Alkoholen, nichtionogenen Emulgatoren und fettlöslichen pharmazeutischen oder kosmetischen Wirkstoffen.

Bei dem fertigen Endprodukt bildet die organische Phase im allgemeinen die innere Phase und die wäßrige Phase die äußere Phase.

2. Verfahrenstechnik

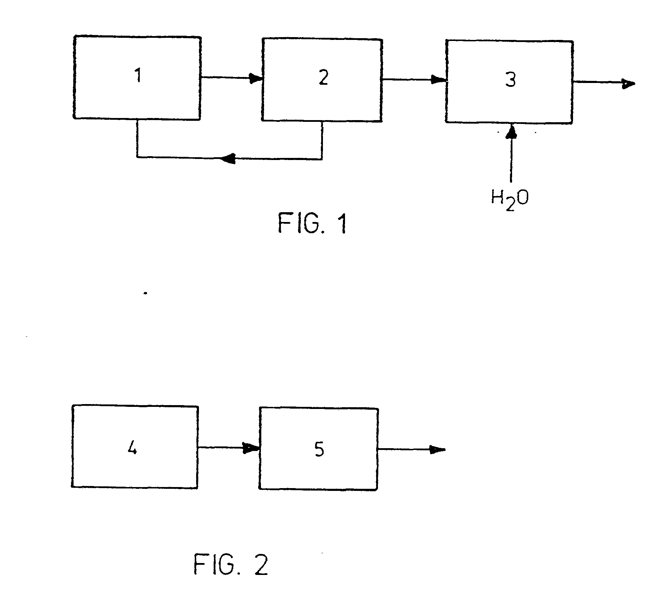

Das erfindungsgemäße Verfahren kann sowohl kontinuierlich als auch diskontinuierlich (Schargenprozeß) durchgeführt werden. Die Figuren 1 und 2 zeigen zwei Varianten für einen Schargenprozeß. Nach Figur 1 wird die organische Phase zusammen mit 5 bis 40 Vol.-% der wäßrigen Phase aus einem Vorratskessel 1 in eine Dispergiermaschine 2 gesaugt oder gepumpt, auf die gewünschte Teilchengröße bei Temperaturen von 55 bis 60°C emulgiert und in den Vorratskessel 1 rezirkuliert. Die Durchsatz- bzw. Kreislaufmengen werden über Rotamesser und Ventile eingestellt. Nach der Dispergierung wird das Emulsionskonzentrat in den Verdünnungskessel 3 überführt, in dem sich der Hauptanteil der wäßrigen Phase mit einer Temperatur von 20°C befindet. Man erhält in der Regel ohne weiteres Rühren ein homogenes Endprodukt.

Bei der Variante nach Figur 2 erfolgt die Dispergierung in einem Rührwerkskessel 4, der gleichzeitig der Ansatzkessel ist: Nach der Dispergierung und Homogenisierung wird das Emulsionskonzentrat in einen Lagertank 5 abgelassen, in dem sich die kalte wäßrige Phase befindet. Es entsteht ebenfalls ein homogenes Endprodukt.

Die Dispergierung erfolgt mit bekannten Verfahren und mit üblichen Emulgiermaschinen wie z. B.

- durch kontinuierliche Direktemulgierung, bei der die zu emulgierenden Phasen mit geregelten Massenströmen kontinuierlich einer Emulgiermaschine zugeführt und homogenisiert werden,
- durch Vorlegen der wäßrigen Phase in einem Kessel, Einemulgieren der organischen Phase und anschließende Homogenisierung,
- durch Vorlegen der organischen Phase in einem Kessel, Einemulgieren der wäßrigen Phase, Phasenuimkehr und anschließende Homogenisierung.

Erfindungsgemäß werden Hochdruck-, Ultraschall- und Düsenhomogenisatoren sowie Rotor/Statordispergiermaschinen verwendet.

Die Teilchengröße bei der Dispergierung kann durch Lichtmikroskopie oder allein mit Hilfe der Ultraschallabsorption und/oder der Streulichtspektroskopie bestimmt werden.

**Beispiel 1**

Mit einer Dispergiermaschine nach dem Rotor/Stator-Prinzip wurden die *organische Phase* (Fettphase), bestehend aus 100 bis 180 kg Ölsäuredecylester, 0,5 bis 2 kg Entschäumer, 0,1 bis 0,2 kg Antioxidantien, 20 bis 80 kg Emulgator, wie Cetylstearylalkohol und Natrium-Cetylstearylsulfat + nichionogene Emulgatoren und die *wäßrige Phase*, bestehend aus 100 bis 300 kg Wasser, 10 bis 60 kg UV-Filtersubstanz - z. B. Novantisolsäure mit Kochsalzzusatz, und 0,5 bis 1,5 kg Konservierungsmittel nach dem Verfahren gemäß Figur 1 angesaugt, emulgiert und in den Ansatzkessel 1 rezirkuliert. Die Durchsatz- bzw. Kreislaufmengen wurden über Rotamesser und Ventile jeweils auf ca. 350 kg/h eingestellt. Die Emulsionstemperatur betrug 50 bis 65°C. Nachdem die organische Phase (Fettphase) vollständig in den Ansatzkessel 1 überführt war, wurden noch 20 bis 40 kg Parfumöl in das Emulsionskonzentrat eingerührt und dann das Emulsionskonzentrat mit einem Mengendurchsatz von etwa 2000 kg/h mittels der Dispergiermaschine 2 in 1000 bis 1200 kg Verdünnungswasser von 20°C in den Verdünnungskessel 3 gedrückt. Es entstand eine Sonnenschutzmilch mit einem hohen Dispersitätsgrad.

**Beispiel 2**

In einem mit einem Käfigrührer ausgestatteten Salbenkessel 4 wurde die Fettphase (organische Phase), bestehend aus 2 bis 4 kg Ölsäuredecylester, 2 bis 5 kg Isopropylmyristat, 3 bis 6 kg Paraffinöl, 0,5 bis 1,1 kg Polyoxiether-Stearinsäureester und 0,5 bis 1,2 kg Sorbitan-Fettsäureester bei einer Temperatur von 60°C vorgelegt und 7 bis 15 kg der wäßrigen Phase bei 20°C, bestehend aus 15 kg nicht neutralisierter Carboxylvinylpolymerisatlösung und 0,2 bis 0,8 kg Paraffinöl dazugegeben. Aus der anfänglichen Wasserin-Fett-Emulsion entstand durch Rühren und Homogenisieren mittels des Käfigrührers eine Fettin-Wasser-Emulsion. Bei diesem Prozeß erfolgt also eine Phasenumkehr, d.h. die anfänglich als äußere Phase vorliegende Wasserphase wird beim Dispergieren zur inneren Phase.

Nach 15 Minuten Homogenisierzeit bei 40°C hat das Emulsionskonzentrat den gewünschten Dispersitätsgrad erlangt und wird in einen gerührten Lagertank 5, in dem 40 bis 60 kg Wasser, 0,1 bis 0,5 kg Alantoin und 20 bis 30 kg nicht neutralisierte Carboxylvinyl-Polymerisatlösung (= insgesamt 0,3 bis 0,8 kg Carbopol in der Zubereitung) vorgelegt sind, abgelassen und mit 0,2 bis 0,6 kg Neutralisationsmittel, wie Triethanolamin oder Natronlauge neutralisiert. Das homogene Endprodukt stellt eine kosmetische Emulsion dar.

**Patentanspruch**

Verfahren zur Herstellung von feinteiligen, stabilen, pharmazeutischen und kosmetischen Dispersionen aus einer wäßrigen Phase und einer in Wasser unlöslichen oder nicht vollständig löslichen organischen Phase, dadurch gekennzeichnet, daß man eine Phasenumkehr während der Dispergierung bei 40 bis 180°C in nur 5 bis 40 Vol.-% der äußeren Phase der fertigen Dispersion unter Verwendung von Hochdruck, Ultraschall- oder Düsenhomogenisatoren oder Rotor/Statordispergiermaschinen und unter Beibehaltung der Konzentrationen von wäßriger und organischer Phase erreicht und dann ohne Veränderung der Teilchengröße mit 60 bis 95 % der äußeren Phase verdünnt, deren Temperatur unter dem Erstarrungspunkt der inneren Phase liegt.

**Claim**

Process for the preparation of fine-particled, stable pharmaceutical and cosmetic dispersions from an aqueous phase and an organic phase which is insoluble or not completely soluble in water, characterised in that a reversal of phases is achieved during the dispersion at 40 to 180°C in only 5 to 40 % by volume of the outer phase of the final dispersion using high-pressure, ultrasonic or jet homogenisers or rotor/stator dispersing machines and while maintaining the concentrations of the aqueous and organic phase, followed by dilution, without changing the particle size, with 60 to 95 % of the outer phase, the temperature of which is below the solidification point of the inner phase.

**Revendication**

Procédé de fabrication de dispersions pharmaceutiques et cosmétiques stables finement divisées à partir d'une phase aqueuse et d'une phase insolubles ou non complétement soluble dans l'eau, caractérisé en ce qu'on réalise une inversion des phases au cours de la dispersion à 40 - 180°C dans seulement 5 à 40 % en volume de la phase externe de la dispersion terminée avec emploi d'homogénéiseurs haute pression, ultrasoniques ou à tuyères ou de machines de dispersion à rotor/stator et tout en respectant les concentrations de la phase aqueuse et de la phase organique, et en ce que, sans modification de la dimension de particule, on dilue avec 60 à 95 % de la phase externe dont la température se situe au-dessous du point de solidification de la phase interne.

FIG. 1

FIG. 2